# EUROPEAN PATENT APPLICATION

(11) **EP 1 972 260 A1**
(43) Date of publication of application: **24.09.2008**
(21) Application number: 08002298.1
(22) Date of filing: 07.02.2008
(51) Int. Cl.: A61B 1/04

(54) **Electronic endoscope apparatus**

(30) Priority: 23.03.2007 JP 2007075936
(71) Applicant: Fujinon Corporation, Saitama-shi, Saitama (JP)
(72) Inventor: Higuchi, Mitsuru, Saitama-shi Saitama (JP); Matsumoto, Kazutoshi, Saitama-shi Saitama (JP)
(74) Representative: Klunker . Schmitt-Nilson . Hirsch

(57) **Abstract**

An electronic endoscope apparatus has: an image forming function of forming a moving image and a still image of an observation object according to an output signal of an image pickup device; and a main electronic enlargement function of forming an electronically enlarged image of the observation object at an optional magnification according to the output signal of the image pickup device in response to an enlargement operation, wherein the electronic endoscope apparatus displays images of the observation object on a main-screen and a subscreen, which are set on a screen of a same monitor, respectively, and wherein the electronic endoscope apparatus comprises a moving-image-for-subscreen generating circuit that forms a moving image which is not electrically enlarged by the main electronic enlargement function and which is converted into a reduced moving image for the subscreen, and when an enlarged still image formed by the electronic enlargement function is displayed on the main-screen, the moving image obtained by reduction conversion at the moving-image-for-subscreen generating circuit is controlled to be displayed on the subscreen.

## Description

### Background of the Invention

### 1. Field of the Invention

The present invention relates to an electronic endoscope and, more particularly, to control the formation and the display of images in an electronic endoscope apparatus enabled to display a moving image, a still image, and an electronically enlarged image of an object on a main-screen and a subscreen set on a monitor.

### 2. Description of the Related Art

Hitherto, electronic endoscope apparatuses have been used for observing organs, such as digestive organs, in a body cavity. In such an electronic endoscope apparatus, an object illuminated with light is imaged by a solid-state image pickup device, for example, a charge coupled device (CCD), mounted in an end portion of an electronic endoscope (scope). Image pickup signals are supplied from the CCD to a processor unit. Predetermined signal processing is performed on the image pickup signals. Thus, an observed object image is displayed on a monitor. Amoving image of an observed obj ect is displayed on this monitor. Also, a still image thereof is formed and displayed on this monitor by operating a freeze switch (still image formation button) . This still image is recorded in a recording apparatus, such as a hard copy apparatus and a digital filing apparatus, by operating a release switch (recording trigger).

In such an electronic endoscope apparatus, a main-screen area and a subscreen area are set on the same monitor. When a still image is displayed on a main-screen, a moving image of an observed object is displayed on a subscreen.

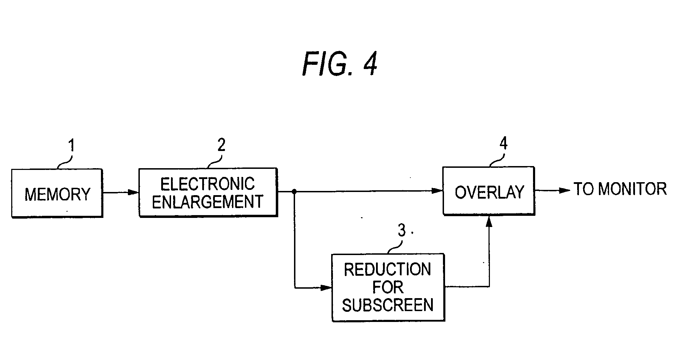

FIG. 4 illustrates the configuration of circuits for forming an image on a main-screen of a related electronic endoscope apparatus. An image memory 1 for storing image data on which predetermined image processing is performed, an electronic enlargement circuit 2 for enlarging an image at an optional magnification according to an enlargement operation and a reduction operation of the enlargement switch, a reduction-for-subscreen circuit 3 for receiving image signals from the electronic enlargement circuit 2 and for reducing the size of an image represented by the received image signal to the size of a subscreen (i.e., reduction conversion of the image represented by the received image signal to an image for the subscreen), and an overlay circuit 4 for overlaying an image for the subscreen, which is output from the image-for-subscreen circuit 3, on an image for the main-screen output from the electronic enlargement circuit 2.

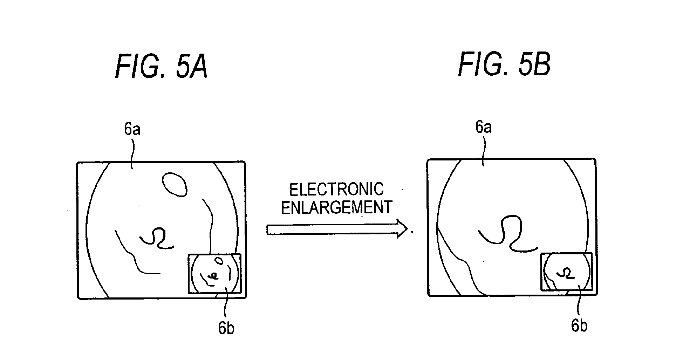

With this configuration, usually, a moving image of an observed object is displayed using the entire screen of the monitor. When the freeze switch is operated, a still image of the observed object is displayed on a main-screen 6a in the entire screen of the monitor, as illustrated in FIG. 5A. Additionally, a moving image of the observed object is displayed in reduced size on a subscreen 6b set in, for example, a rightwardly lower part of the entire screen of the monitor, as viewed in FIG. 5A. Subsequently, when an enlargement operation is performed by operating the enlargement switch, image enlargement processing is performed in the electronic enlargement circuit 2. As illustrated in FIG. 5B, an enlarged still image is displayed on the main-screen 6a, while an enlarged moving image is displayed on the subscreen 6b. The still image shown in FIG. 5A and the enlarged still image shown in FIG. 5B are recorded in the recording apparatus by operating the release switch.

However, in the related electronic endoscope apparatus, as described with reference to FIG. 5B, when the enlargement switch is enlarged according to an operation performed on the enlargement switch, an image displayed on the subscreen 6b is an enlarged image (moving image). Thus, the related endoscope apparatus has a problem in that a state of a large range of an observed object is difficult to know. That is, a still image at the time point of operating the freeze switch is displayed on the main-screen 6a, while a moving image at a current time point on the subscreen 6b is displayed on the subscreen 6b. Therefore, a non-enlarged image of an observed object, in which a large range of the object can be observed, is preferable to an enlarged image thereof, for checking and knowing a current state of an observed object on the subscreen.

### Summary of the Invention

The invention is accomplished in view of the aforementioned problems. An object of the invention is to provide an electronic endoscope apparatus that enables a user to favorably observe and know a state of an observation object by displaying a non-electronically-enlarged moving image of the object on a subscreen even in a case where an image displayed on a main-screen is electronically enlarged by performing an enlargement operation.

To achieve the foregoing object, according to the invention, there is provided an electronic endoscope apparatus (hereunder referred to as a first electronic endoscope apparatus), having: an image forming function of forming a moving image and a still image of an observation object according to an output signal of an image pickup device; and a main electronic enlargement function (i.e., electronic-enlargement-for-main-screen function) of forming an electronically enlarged image of the observation object at an optional magnification according to the output signal of the image pickup device in response to an enlargement operation, wherein the electronic endoscope apparatus displays images of the observation object on a main-screen and a subscreen, which are set on a screen of the same monitor, respectively. The first electronic endoscope apparatus comprises a moving-image-for-subscreen generating circuit that forms a moving image which is not electrically enlarged by the main electronic enlargement function and which is converted into a reduced moving image for the subscreen. When an enlarged still image formed by the electronic enlargement function is displayed on the main-screen, the moving image obtained by reduction conversion at the moving-image-for-subscreen generating circuit is controlled to be displayed on the subscreen.

In an embodiment (hereunder referred to as a second electronic endoscope apparatus) of the first electronic endoscope apparatus, when a freeze operation is performed so as to form a still image of the observation object, an enlarged still image formed by the main electronic enlargement function is displayed on the main-screen, while a moving image obtained by reduction conversion performed at the moving-image-for-subscreen generating circuit is displayed on the subscreen.

In an embodiment (hereunder referred to as a third electronic endoscope apparatus) of the first or second electronic endoscope apparatus, the electronic endoscope apparatus further has an auxiliary electronic enlargement function (i.e., electronic-enlargement-for-subscreen function) of enlarging the moving image, which is obtained by the reduction conversion for the subscreen, at a fixed magnification set for the subscreen, and the moving image enlarged at the fixedmagnification for the subscreen is displayed on the subscreen.

With the aforementioned configuration, when, for example, a freeze operation is performed, a still image is displayed on the main-screen, instead of a moving image. A moving image is displayed on the subscreen that appears at, for example, a rightwardly lower part of the screen of the same monitor. When an enlargement operation is subsequently performed, a still image enlarged by the main electronic enlargement function is displayed on the main-screen. On the other hand, a non-enlarged moving image is continuously displayed on the subscreen. When a release operation is performed, an enlarged still image is recorded in the recording apparatus. In a case where no freeze operation is performed, and where an enlarged operation is solely performed, the electronic endoscope apparatus can be adapted so that an enlarged moving image is displayed on the main-screen, and that a non-enlarged moving image is displayed on the subscreen that has appeared in the main-screen.

According to the third electronic endoscope apparatus, a moving image having undergone the reduction conversion is enlarged at a fixed magnification by the auxiliary electronic enlargement function. The size of the subscreen is maintained. Then, a moving image obtained by slightly enlarging a moving image (image itself), which is represented by signals output from a CCD, is displayed on the subscreen. That is, a fixed magnification for enlarging a moving image on the subscreen is set in a setting window, such as a user menu, which is displayed by a processor unit, using a keyboard or the like. Consequently, the size of a moving image displayed on the subscreen can be enlarged at an optional magnification of, for example, 1.4, 2, ..., at which an object can easily be observed.

### Brief Description of the Drawings

FIG. 1 is a circuit diagram illustrating the configuration of an electronic endoscope apparatus according to an embodiment of the invention;
FIG. 2 is a diagram illustrating a state of display on a monitor in a case where a still image display operation, an electronic enlargement operation, and a still image recording operation are performed in this order according to the embodiment;
FIG. 3 is a diagram illustrating a state of display on a monitor in a case where an electronic enlargement operation and a still image display operation are performed in this order according to the embodiment;
FIG. 4 is a circuit diagram illustrating the configuration of a circuit for forming images on a main-screen and a subscreen on a monitor of the related-art electronic endoscope apparatus; and
FIG. 5 is a diagram illustrating a state of display on a monitor in a case where an electronic enlargement operation is performed according to the embodiment after a still image display operation.

### Detailed Description of the Invention

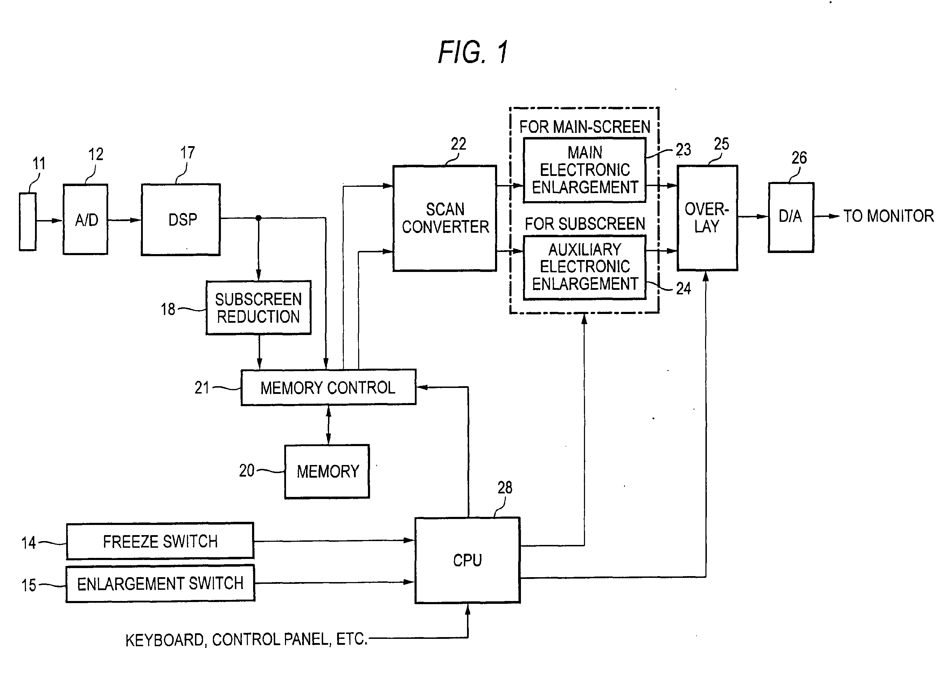

FIG. 1 illustrates the configuration of an electronic endoscope according to an embodiment. This electronic endoscope apparatus includes an electronic endoscope (i.e., an electronic scope), a processor unit, and a monitor. As illustrated in FIG. 1, a CCD (i.e., a solid-state image pickup device) 11 and an analog-to-digital converter 12 for performing analog-to-digital conversion on image pickup signals output from the CCD 11 are mounted in the electronic endoscope. A freeze switch 14 for operating both the display (corresponding to the depression of a first-stage of the freeze switch 14 or to a first depression of the freeze switch 14) and the recording (corresponding to the depression of a second-stage of the freeze switch 14 or to a second depression of the freeze switch 14) of a still image, and an enlargement switch 15 for handling an electronic enlargement magnification are provided in an operation portion of this electronic endoscope. The recording of the still image can be operated using a release (or recording) switch disposed separately from a freeze switch 14.

On the other hand, for example, a digital signal processor (DSP) 17 and subsequent circuits are provided in the processor unit. The DSP circuit 17 performs various kinds of image processing on image signals (video signals) output from the A/D converter 12. Also, a reduction-for-subscreen circuit 18 for performing reduction conversion on an image obtained by the DSP circuit 17 (i.e., reducing this image in size to an image to be displayed on the subscreen), an image memory 20 for storing image data representing moving images and still images, and a memory control circuit 21 are provided subsequent to the DSP circuit 17.

Additionally, a scan converter 22 for converting the number of scanning lines represented by or the horizontal synchronizing frequency of image signals output from the image memory 20, a main electronic enlargement circuit 23 for enlargement of images (including moving images and still images) to be displayed on the main-screen (incidentally, the enlargement includes a case where an enlarging magnification = 1.0), and an auxiliary electronic enlargement circuit 24 for enlarging a moving image to be displayed on the subscreen (incidentally, this enlargement includes the case where an enlarging magnification = 1.0) are provided as circuits for implementing the electronic enlargement function. Furthermore, an overlay circuit 25 for overlaying an image displayed on the main-screen, which is output from the main electronic enlargement circuit 23, with an image displayed on the subscreen output from the auxiliary electronic enlargement circuit 24 according to, for example, a picture-in-picture method, and a digital-to-analog (D/A) converter 26 for converting a digital signal output from the overlay circuit 25 into an analog signal are provided. Output signals of the D/A converter 26 is supplied to the monitor. Incidentally, other methods, such as a picture-on-picture method and a superimposing method, can be employed as the method of overlaying an image displayed on the main screen with an image displayed on the subscreen.

Acentral processingunit (CPU) or amicrocomputer 28 is provided as a circuit for integrated control of the above circuits. The CPU 28 controls the display and the recording of still images according to manipulate-signals for the freeze switch 14, the electronic enlargement at a given magnification accordingto manipulate-signals for the enlargement switch 15, the setting of a fixed magnification for a moving image to be displayed on the subscreen, and the enlargement of amoving image at the fixedmagnification. An optional magnification value can be selected in a fixed-magnification setting window, which is provided in the user menu, for a moving image to be displayed on the subscreen as the fixed magnification corresponding to the moving image to be displayed thereon, using a keyboard provided in the processor unit and a control panel. This fixed magnification differs from a large magnification (whose value is equal to or more than several tens) to be set using the enlargement switch 15, and has a small magnification (e.g., at most several times) to the extent that the visualizationof amoving image displayed on the subscreen is facilitated.

With the above-described configuration of the present embodiment, when a power supply for the apparatus is turned on, an observation object is imaged by the CCD 11 shown in FIG. 1. Subsequently, an image signal processed by the DSP 17 is stored once in the image memory 20. Thereafter, a moving image of an observation object is displayed on the screen of the monitor 30, without displaying the subscreen, by outputting this image signal from the D/A converter 26 to the monitor.

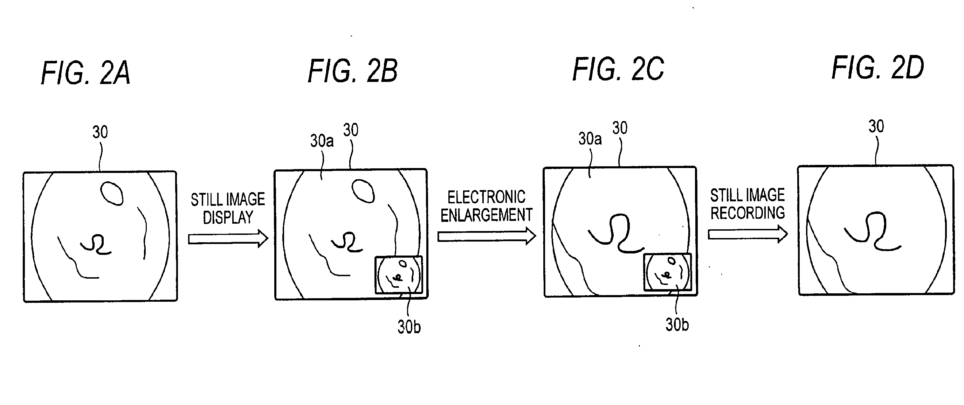

Then, at the depression of the first-stage of the freeze switch 14 or at the first depression of the freeze switch 14, the CPU 28 performs a control operation so as to display a still image. Thus, an image signal output from the DSP circuit 17 is stored in a still image memory portion of the image memory 20 as a still image signal representing a still image to be displayed on the main-screen. Thereafter, the still image signal is read from the image memory 20 is supplied to the overlay circuit 25. Simultaneously with this, a moving image signal is read from the image memory 20. Then, the reduction-for-subscreen circuit 18 performs reduction conversion on a moving image represented by the read moving image signal to an image having a size fit to that of the subscreen. A moving image signal representing the image obtained by the conversion is once stored in the image memory 20. Thereafter, this moving image signal is supplied to the overlay circuit 25. Then, the overlay circuit 25 overlays a still image represented by the still image signal with a moving image represented by the moving image signal according to a picture-in-picture method. Consequently, as illustrated in FIG. 2B, a still image is displayed on the main-screen 30a of the monitor 30. A moving image is displayed on the subscreen 30b placed at a rightward lower part of the main-screen 30a, as viewed in FIG. 2B.

Subsequently, when an electronic enlargement operation is performed using the enlargement switch 15, a still image represented by a still image signal, which read from the image memory 20, is enlarged through the scan converter 22 and the main electronic enlargement circuit 23 at the magnification set by the enlargement operation. The overlay circuit 25 overlays the enlarged still image represented by an enlarged still image signal with an image represented by the moving image signal to be displayed on the subscreen. Consequently, as illustrated in FIG. 2C, the enlarged still image is displayed on the main-screen 30a, while the moving image, which has the same size as that of the moving image shown in FIG. 2B and covers a wide field of view, is displayed on the subscreen 30b placed at the rightward lower part of the main-screen 30a. That is, even in a case where a still image displayed on the main-screen 30a is enlarged, no moving image is displayed on the main-screen 30a. The moving image, which has the same size as that of the moving image shown in FIG. 2B and covers a wide field of view, is displayed on the subscreen 30b. Subsequently, when a recording (or release) operation is performed by the depression of the second stage (or the second depression) of the freeze switch 14, an enlarged still image shown in FIG. 2D is recorded and held in the recording apparatus.

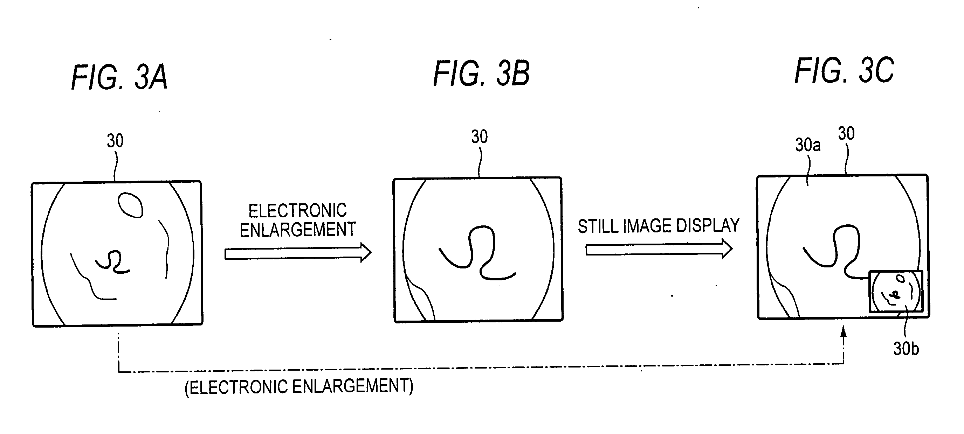

Referring to FIGS. 3A to 3C, when an electronic enlargement operation is performed using the enlargement switch 15, in a state illustrated in FIG. 3A, in which a moving image is displayed on the main-screen, an image electronically enlarged at a magnification set by the enlargement operation is displayed on the screen of the monitor 30, without appearance of the subscreen, according to an electronic enlargement control operation performed by the CPU 28, as illustrated in FIG. 3B. Subsequently, when the first stage of the freeze switch 14 is depressed, an enlarged still image is displayed on the main-screen 30a, while a non-enlarged moving image is displayed on the subscreen, as illustrated in FIG. 3C. That is, according to the present embodiment, when a sill image is displayed by operating the freeze switch 14, the subscreen 30b, on which a moving image is displayed, is caused to appear. A current state of the observation object shown in the still image can be checked according to this moving image.

This moving image can be displayed on the subscreen 30b not only in the case of displaying the still image on the main-screen 30a but in the case of displaying an electronically enlarged image thereon. That is, when the enlargement switch 15 is operated in the state illustrated in FIG. 3A, the CPU 28 can control the state of the screen to be changed to the state illustrated in FIG. 3C, so that the subscreen 30b, on which a moving image is displayed, appears in the rightwardly lower part of the main-screen 30a on which the enlarged still image is displayed.

Additionally, the present embodiment can be modified so that the size of a moving image to be displayed on the subscreen 30b is changed while the size (or area) of the subscreen 30b is maintained. In this case, as described above, the fixed magnification, at which a moving image is displayed on the subscreen 30b, is preliminarily set in the setting window provided in the user menu to be displayed on the monitor 30, using, for example, the keyboard provided in the processor unit. Then, the CPU 28 controls the enlargement of the moving image, which is performed by the auxiliary electronic enlargement circuit 24. Thus, the moving image displayed on the subscreen 30b is enlarged at the set magnification. Consequently, a moving image, which meets the conditions for an observed part and requests from an operator and which covers a wide field of view, can be observed on the subscreen 30b.

In the electronic endoscope according to the invention, a non-electrically enlarged moving image is displayed on the subscreen even in a case where an image displayed on the main-screen is electronically enlarged at an optional magnification by an enlargement operation. Thus, a moving image covering a field of view wider than an image of an observed object displayed on the main-screen can visuallybe checked on the subscreen. Consequently, a state of the observed object can preferably be observed and known.

According to the third electronic endoscope apparatus, a fixed magnification for displaying an image on the subscreen can preliminarily optionally be set, independent of enlargement of an image displayed on the main-screen. The size of a moving image displayed on the subscreen can be adjusted to preferably meet conditions for an observed part, observations, operations and requests from operators. Thus, the third electronic endoscope apparatus has advantages in facilitating the observation and the prehension of an observed object.

The entire disclosure of each and every foreign patent application from which the benefit of foreign priority has been claimed in the present application is incorporated herein by reference, as if fully set forth.

## Claims

1. An electronic endoscope apparatus having: an image forming function of forming a moving image and a still image of an observation object according to an output signal of an image pickup device; and a main electronic enlargement function of forming an electronically enlarged image of the observation object at an optional magnification according to the output signal of the image pickup device in response to an enlargement operation,
wherein the electronic endoscope apparatus displays images of the observation object on a main-screen and a subscreen, which are set on a screen of a same monitor, respectively, and
wherein the electronic endoscope apparatus comprises a moving-image-for-subscreen generating circuit that forms a moving image which is not electrically enlarged by the main electronic enlargement function and which is converted into a reduced moving image for the subscreen, and
when an enlarged still image formed by the electronic enlargement function is displayed on the main-screen, the moving image obtained by reduction conversion at the moving-image-for-subscreen generating circuit is controlled to be displayed on the subscreen.

2. The electronic endoscope apparatus according to claim 1,
wherein, when a freeze operation is performed so as to form the still image of the observation object, an enlarged still image formed by the main electronic enlargement function is displayed on the main-screen, while a moving image obtained by the reduction conversion performed at the moving-image-for-subscreen generating circuit is displayed on the subscreen.

3. The electronic endoscope apparatus according to claim 1, further having an auxiliary electronic enlargement function of enlarging the moving image, which is obtained by the reduction conversion for the subscreen, at a fixed magnification set for the subscreen, and
wherein the moving image enlarged at the fixed magnification for the subscreen is displayed on the subscreen.

4. The electronic endoscope apparatus according to claim 2, further having an auxiliary electronic enlargement function of enlarging the moving image, which is obtained by the reduction conversion for the subscreen, at a fixed magnification set for the subscreen, and
wherein the moving image enlarged at the fixed magnification for the subscreen is displayed on the subscreen.
